# EUROPÄISCHE PATENTSCHRIFT

(11) **EP 2 846 732 B1**
(45) Veröffentlichungstag und Bekanntmachung des Hinweises auf die Patenterteilung: **04.07.2018**
(21) Anmeldenummer: 13722351.7
(22) Anmeldetag: 03.05.2013
(51) Int. Cl.: A61F 2/07

(54) **INTRALUMINALE GEFÄSSPROTHESE MIT IN-SITU-FENESTRIERUNG**
IN-SITU FENESTRATED INTRALUMINAL VASCULAR PROSTHESIS
PROTHÈSE VASCULAIRE INTRALUMINALE À FENESTRATION IN SITU

(30) Priorität: 07.05.2012 DE 102012103985
(43) Veröffentlichungstag der Anmeldung: 18.03.2015
(73) Patentinhaber: JOTEC GmbH, 72379 Hechingen (DE)
(72) Erfinder: BARTHOLD, Franz-Peter, 72336 Balingen (DE)
(74) Vertreter: Witte, Weller & Partner Patentanwälte mbB
(86) Internationale Anmeldenummer: PCT/EP2013/059275
(87) Internationale Veröffentlichungsnummer: WO 2013/167493

(56) Entgegenhaltungen:
- WO-A1-2009/056644
- US-A1- 2001 047 198
- US-A1- 2010 161 025
- US-B1- 6 689 162

## Beschreibung

Die vorliegende Erfindung betrifft eine intraluminale Gefäßprothese zur Implantation in ein Blutgefäß eines Patienten, mit in ihrer Längsrichtung hintereinander angeordneten Ringen aus mäanderförmig umlaufenden Stützen und einem an den Ringen befestigten und diese verbindenden Prothesenmaterial, wobei das Prothesenmaterial einen hohlzylindrischen Körper mit umfänglich geschlossenem Mantel bildet, und wobei das Prothesenmaterial eine Gewebestruktur mit einer Maschendichte aufweist.

Insbesondere betrifft die vorliegende Erfindung intraluminale Gefäßprothesen, die zur in-situ-Ausbildung von Fenestrierungen im Prothesenmaterial geeignet sind, sowie Verfahren zur Ausbildung von Fenestrierungen in intraluminalen Gefäßprothesen.

Allgemein ist es bekannt, intraluminale Gefäßprothesen, die auch als endovaskuläre Gefäßstents/-stentgrafts bezeichnet werden, zur Behandlung von Aneurysmen in Arterien zu implantieren. Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen oder, wie bei dem sog. falschen Aneurysma bzw. der sog. Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einem Ruptur der Arterie führen mit der Folge, dass der Patient innerlich verblutet.

Zur Behandlung von Aneurysmen ist es bereits bekannt, die betroffene Arterie durch Implantation eines Stents/Stentgrafts zu stabilisieren, um eine Ruptur des Gefäßes zu vermeiden.

Die zur Behandlung von Aneurysmen verwendeten Stents/Stengrafts bestehen dabei im Allgemeinen aus einem röhrchenförmigen Metallrahmen, dessen Mantelfläche mit einer Textil- oder Polymerfolie abgedeckt ist, so dass sich ein hohlzylindrischer Körper ergibt. Zur Implantation wird der Stent - bspw. mittels einer diesen umgebenden und komprimierenden Hülle - radial zusammengedrückt, so dass sich seine Querschnittsfläche deutlich verringert. Der Stent/Stentgraft wird dann mit Hilfe eines Einführsystems in den Bereich des Aneurysmas gebracht, wo der Stent freigesetzt wird. Aufgrund der Federwirkung des Metallrahmens expandiert der Stent wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch den Stent/Stentgraft, wodurch eine weitere Belastung der Aussackung verhindert wird.

Die Expansion des Metallrahmens kann einerseits durch die Verwendung von selbstexpandierendem Metall, wie bspw. Nitinol, bewirkt werden, oder aber durch Einsatz eines Dilations-Ballons, der von innen in den Metallrahmen eingeführt wird und durch dessen Dilatation auch der Metallrahmen expandiert wird.

Oftmals zweigen an der Stelle des Gefäßes, an welcher ein solcher Stent/Stentgraft bzw. eine solche Gefäßprothese eingeführt werden soll, SeitenBlutgefäße ab, weshalb bei Einbringung des Gefäßimplantats dann die Gefahr besteht, dass diese Seitengefäße durch die Gefäßprothese im Hauptgefäß bzw. durch das blutdichte Prothesenmaterial von der Blutzufuhr abgeschnitten werden. Daher weisen Gefäßprothesen in diesen Bereichen oftmals Öffnungen, sog. "Fenestrierungen", im Mantel- bzw. Prothesenmaterial auf, um das durch die Gefäßprothese fließende Blut durch diese Öffnungen auch in die vom Gefäß abzweigenden Seiten-Gefäße zu lenken. Dadurch wird auch eine Blutversorgung der Körperbereiche, die von den Seite-Gefäßen versorgt werden, gewährleistete.

Die an solchen Abzweigungs-Bereichen einzubringende Gefäßimplantate weisen in vielen Fällen selber noch vom Gefäßprothesen-Grundkörper abzweigende Seitenäste auf, die in de Seiten-Gefäße hineinreichen. Dadurch wird zusätzlich sicher gestellt, dass auch die Seiten-Gefäße mit Blut versorgt werden.

So ist bspw. aus der US 2008/0312732 A1 ein Stentgraft bekannt, der eine röhrenförmige Wand mit zumindest einer Fenestrierung und einer peripheren Verstärkung um zumindest einen Teil der Fenestrierung herum aufweist. Der Stengraft kann ferner röhrenförmige "Extensionen" aufweisen, also Seitenärmchen, die sich von dem Grundkörper des Stentgrafts erstrecken und die mit dem Stentgraft über die Fenestrierung in Fluid-Kommunikation stehen.

Ferner ist aus der WO 2009/064672 A2 eine Vorrichtung und ein Verfahren zur in-situ-Stengraft-Fenestrierung bekannt, bei welchem der Haupt-Stentgraft in situ, d.h. also nach im Gefäß erfolgter Positionierung, mit einer Nadel penetriert wird, um ein Nadelloch im Graft-Material zu bilden. Anschließend wird eine Dilator-Anordnung durch das Nadelloch geschoben, um das Nadelloch zu erweitern. Die WO2009/056644 A1 offenbart eine Prothese aus einem gewebten Graft mit verstärkten Fenestrierungsbereichen. Die US2010/0161025 A1 offenbart einen Stent-Graft mit Fenestrierungsbereichen mit verringerter Dichte und in den Stent-Graft eingewebten Metallfäden.

Nachteile der im Stand der Technik bekannten Stentgrafts für die in-situ-Fenestrierung bestehen darin, dass sie sehr korrekt in Bezug auf die abzweigenden Gefäße positioniert werden müssen, und insbesondere darin, dass das Graft-Material an vorgesehenen Positionen zerrissen bzw. verletzt wird, um die Fenestrierungen zu bilden. Dadurch besteht die Gefahr des weiteren Ausreißens des Graft-Materials, so dass sich die Fenestrierung unkontrolliert erweitert, wodurch es als Folge wiederum zu einem unkontrollierten Ausfließen des Blutes in diesem Bereich der Gefäßprothese kommen kann. Daher ist es bei den im Stand der Technik bekannten Gefäßimplantaten für die in-situ-Fenestrierung meist notwendig, ein auf den jeweiligen Patienten bzw. dessen Gefäß genau zugeschnittenes Gefäßimplantat bereitzustellen, wenn eine erfolgreiche Behandlung erreicht werden soll.

Aufgabe der vorliegenden Erfindung ist es daher, eine intraluminale Gefäßprothese bzw. einen Stentgraft bereitzustellen, mit dem die oben geschilderten Nachteile überwunden werden können, und mit welchen Gefäßimplantate bereitgestellt werden können, die flexibel, d.h. nicht maßgeschneidert eingesetzt werden können, wobei gleichzeitig die Dimensionen der Fenestrierungen gewährleistet bleiben.

Die Erfindung ist durch die Ansprüche definiert. Erfindungsgemäß wird diese Aufgabe durch eine intraluminale Gefäßprothese erreicht, die in Längsrichtung hintereinander angeordnete Ringe aus mäanderförmig umlaufenden Stützen und ein an den Ringen befestigtes und diese verbindende Prothesenmaterial mit dieser Gewebestruktur aufweist, welches Prothesenmaterial einen hohlzylindrischen Grundkörper mit umfänglich geschlossenem Mantel bildet, wobei die Gewebestruktur des Prothesenmaterials aus miteinander verwobenen Fäden gebildet ist und eine Maschendichte aufweist, und wobei zwischen zwei hintereinander angeordneten Ringen der Gefäßprothese im Prothesenmaterial zumindest ein definierter Fenestrierungs-Bereich für die Ausbildung von zumindest einer Öffnung für von den hohlzylindrischen Grundkörper abgehende Seitenäste vorgesehen ist, wobei der Fenestrierungs-Bereich eine zum übrigen Prothesenmaterial unterschiedliche Gewebestruktur aufweist und wobei die Gewebestruktur im Fenestrierungs-Bereich eine geringere Maschendichte aufweist als in den übrigen Prothesenmaterial, sowie gitterartige Verstärkungselemente.

Die erfindungsgemäße Prothese kann ferner verwendet werden in einem Verfahren zur Fenestrierung einer intraluminalen Gefäßprothese, wobei das Verfahren die Schritte des Bereitstellen eines intraluminalen Gefäßprothese wie gerade beschrieben umfasst, sowie den Schritt des Durchdringens des Fenestrierungs-Bereiches der intraluminalen Gefäßprothese mit einem Erweiterungselement, bspw. einem Katheter, einem Dilator oder einem Führungsdraht bzw. deren Spitzen, zur Ausbildung von zumindest einer Öffnung im Fenestrierungs-Bereich, wobei das Prothesenmaterial im Bereich der zu bildenden Öffnung durch die Dilatorspitze verdrängt wird.

Die der Erfindung zugrundeliegende Aufgabe wird auf diese Weise vollkommen gelöst.

Mit der erfindungsgemäßen Gefäßprothese wird dem Anwender der Gefäßprothese die Möglichkeit geschaffen, diese ohne Rücksicht auf bereits festgelegt Fenestrierungen bzw. Fenestrierungs-Bereiche zügig in einem Gefäß freizusetzen, wobei ggf. lediglich darauf geachtet werden muss, dass dann, wenn lediglich ein Fenestrierungs-Bereich vorgesehen ist, sich dieser im Bereich der von dem Hauptgefäß abgehenden Seiten-Gefäße befindet. Der Anwender kann nun in diesem Fenestrierungs-Bereich das Prothesenmaterial, bspw. mit Hilfe eines Katheters, Dilators oder eines Führungsdrahtes oder eines sonstigen geeigneten Elements derart durchdringen, dass das Gewebe nicht zerrissen, zerstört, durchlöchert oder in sonstiger Weise verletzt wird, sondern die Fäden der Gewebestruktur von dem einzusetzenden Element verdrängt werden, so dass sich sozusagen eine große Masche bildet, die die Öffnung oder Fenestrierung für das abgehende Seitengefäß darstellt. Ermöglicht wird dies durch die besondere Gewebestruktur in dem Fenestrierungs-Bereich, nämlich derart, dass hier eine geringere Maschendichte vorgesehen ist, die gleichzeitig durch die gitterartigen Verstärkungselemente strukturiert und definiert wird. Die geringere Maschendichte bewirkt, dass es mit entsprechenden Hilfsmitteln einfach ist, die durch die Fäden gebildete Masche zu vergrößern bzw. allgemein das Material zur Bildung einer Öffnung zu verdrängen. Die gitterförmigen Verstärkungselemente, die erfindungsgemäß nur im Fenestrierungs-Bereich und nicht außerhalb dessen vorgesehen sind, gewährleisten dabei gleichzeitig, dass die gebildete Öffnung nicht ausreißt und dadurch die Gefahr einer unkontrollierten Öffnung entsteht, durch die unkontrolliert Blut entweichen kann.

Entsprechend kann die erfindungsgemäßen Gefäßprothese daher bspw. im Aortenbogen oder im thorakalen oder iliakalen Bereich, mithin also vorzugsweise in allen Gefäßbereichen, die einer Unterstützung bedürfen, und von denen im zu behandelnden Abschnitt Seitenblutgefäße abzweigen, eingesetzt werden.

Vorliegend werden die Begriffe "Öffnung" und "Fenestrierung" auch synonym verwendet.

Die in einem Abstand hintereinander angeordneten Ringe aus mäanderförmig umlaufenden Stützen stellen Stents bzw. Stent-Ringe dar.

Dabei bedeutet vorliegend "Gewebestruktur", dass das Prothesenmaterial der intraluminalen Gefäßprothese aus miteinander verwebten Kettfäden und Schussfäden hergestellt ist. Mit den Begriffe "Kettfäden" und "Schussfäden" sind vorliegend - wie auch üblicherweise im die Erfindung betreffenden Gebiet - die bei Geweben üblicherweise verwendeten beiden Fadensysteme gemeint; zur manuellen oder maschinellen Herstellung gewebter textiler Erzeugnisse werden mindestens zwei Fadensysteme rechtwinklig oder nahezu rechtwinklig verkreuzt. Dabei werden üblicherweise die Fäden in Längsrichtung als Kette oder Kettfäden bezeichnet, und die Querfäden Schuss oder Schussfäden. Die Fäden werden durch Fadenkreuzung verbunden, was nicht bedeutet, dass die Fäden kreuzend aufeinander liegen, sondern dass Fäden in einem bestimmten Rhythmus über und unter den querliegenden Fäden durchgeführt werden.

Üblicherweise werden die Garne, die zur Herstellung von intraluminalen Gefäßprothesen eingesetzt werden, in verschiedenen Richtungen zusammengewebt, bspw. derart, dass ein Satz Kettenfäden in Längsrichtung parallel zu den Webkanten verläuft und dadurch die Breite des gewebten Produktes darstellt, und dass ein Satz Schussfäden dann von Webkante zu Webkante unter einem rechten Winkel zu den Kettenfäden in diese gewebt werden.

Die hohlzylindrische Form der Gefäßprothese wird dann üblicherweise dadurch erreicht, dass das gewebte Prothesenmaterial vernäht und ggf. zugeschnitten wird. So werden bspw. auch die mäanderförmig umlaufenden Ringe jeweils mit dem Prothesenmaterial vernäht, zur Ausbildung der Röhrenform der Prothese.

Gemäß einer bevorzugten Ausführungsform der erfindungsgemäßen intraluminalen Gefäßprothese ist die Gewebestruktur des Prothesenmaterials im Fenestrierungs-Bereich durch eine integrierte Webung hergestellt und das Prothesenmaterial einstückig gewebt und ausgebildet.

Diese Ausführungsform hat den Vorteil, dass die Gefäßprothese an einem Stück gewebt werden kann, wobei der Webeprozess derart ausgebildet bzw. programmiert ist, dass im Fenestrierungs-Bereich eine weniger dichte Maschenstruktur generiert wird als in den übrigen Bereichen des Prothesenmaterials der Gefäßprothese, bzw. über die gesamte Länge der Gefäßprothese hinweg.

Vorliegend wird unter "weniger dichte Massenstruktur" bzw. "geringere Maschendichte als das übrige Prothesenmaterial" verstanden, dass in diesem Bereich, d.h. im Fenestrierungs-Bereich, im Prothesenmaterial eine kleinere Maschendichte vorliegt als in den Bereichen ohne bzw. außerhalb des Fenestrierungs-Bereiches, wobei die kleinere Maschendichte derart ausgestaltet ist, dass die Maschen-bildenden Fäden des Prothesenmaterials - beispielsweise mit Hilfe eines Katheters, Dilators oder Führungsdrahtes - derart verdrängt werden können, dass das Prothesenmaterial nicht zerrissen oder in sonstiger Weise verletzt wird, was in den Bereichen außerhalb des Fenestrierungs-Bereiches der Fall wäre.

Ferner wird vorliegend mit "übriges Prothesenmaterial" dasjenige Prothesenmaterial bezeichnet, das keine Fenestrierungs-Bereiche aufweist, und in dessen Bereichen keine Fenestrierungen vorgesehen werden. In der Regel sind diese Abschnitte auch blutdicht.

Unter "gitterartige Verstärkungselemente" wird vorliegend jede Struktur verstanden, mittels derer ein Muster gebildet werden kann, bei welchem sich längliche Elemente kreuzen und dadurch Überschneidungspunkte der länglichen Elemente sowie dazwischen liegende offene Bereiche gebildet werden. Diese Verstärkungselemente sind zusätzlich zu den bereits die Maschenstruktur des Prothesenmaterials bildenden Textilfäden in und/oder auf das Prothesenmaterial eingearbeitet.

Erfindungsgemäß bilden diese gitterartigen Verstärkungselemente eine Ausrissgrenze, mit der verhindert wird, dass die durch die Verdrängung der Fäden gebildeten größeren Maschen bzw. Öffnungen nicht ausreißen, sondern in einer durch die Gittergröße vorgegebenen Dimension bzw. Grenze verbleiben.

Gemäß einer weiteren bevorzugten Ausführungsform erstreckt sich der Fenestrierungs-Bereich über den vollen Umfang des hohlzylindrischen Grundkörpers. Diese Ausführungsform hat den Vorteil, dass die Gefäßprothese ohne Rücksicht auf die genaue Lokalisierung des Fenestrierungs-Bereiches in Bezug auf den Umfang der Gefäßprothese in dem Gefäß platziert werden kann. Vielmehr hat der Anwender die Möglichkeit, die Gefäßprothese zügig in das zu behandelnde Gefäß einzuführen und dann im Fenestrierungs-Bereich an einer beliebigen Lage im Umfang der Gefäßprothese diese mit einer oder mehreren Öffnungen zu versehen. Die Öffnungen werden dabei je nach Lage der von dem zu behandelnden Blutgefäß ausgehenden Seitengefäße angebracht, ohne dass es notwendig wäre, die Gefäßprothese bereits im Vorfeld in ihrem Umfang hierauf auszurichten, also ohne diese im Gefäß um ihre Achse zu rotieren.

Entsprechend wird vorliegend mit "vollem Umfang des hohlzylindrischen Grundkörpers" der gesamte Umfangsbereich gemeint, den der hohlzylindrische Grundkörper im Fenestrierungs-Bereich besitzt. Vorteilhafterweise kann durch eine entsprechende Webung des gesamten Prothesenmaterials dies einfach realisiert werden, da die Gefäßprothese dann im Fenestrierungs-Bereich über ihren gesamten Umfang eine geringere Maschendichte aufweist, als die übrigen Bereiche des Prothesenmaterials.

Erfindungsgemäß werden die gitterartigen Verstärkungselemente im Fenestrierungs-Bereich durch gitterartig angebrachte zusätzliche - d.h. zu den Textilfäden des Prothesenmaterials zusätzliche - Textilfäden und/oder Klebstoffe bewirkt oder erzielt .

Mit zusätzlichen, gitterartig angebrachten Textilfäden oder Klebstoffen im Fenestrierungs-Bereich wird den zu bildenden Öffnungen, bzw. erweiterten Maschen durch die Gittergröße eine einfache Grenze vorgegeben, innerhalb derer sie aufweiten können. Wird nach der Bildung der Öffnung beispielsweise eine weitere, zweite Gefäßprothese durch die Öffnung in der implantierten Gefäßprothese geschoben, wird die Öffnung dadurch zwar ggf. zusätzlich erweitert, jedoch wird durch das im Prothesenmaterial vorgesehene Gitter, bzw. den einzelnen, durch die Textilfäden/den Klebstoffen vorgegebenen Gitterelemente eine Begrenzung vorgegeben. Mit diesem kann verhindert werden, dass auch nach Einbringen einer zweiten Gefäßprothese durch diese Öffnungen hindurch das Prothesenmaterial der implantierten Gefäßprothese weiter aufreißt und dieses dadurch verletzt wird.

Die verstärkenden Textilfäden/Klebstoffe im Fenestrierungs-Bereich können entweder in diesen Bereich zusätzlich eingewebt werden, oder alternativ wird das Gitter durch auf das Prothesematerial aufzubringende Klebstoffe bewirkt. Bei dieser Alternative wird mittels der Klebstoffe eine Gitterstruktur gebildet, die wie die durch die Textilfäden gebildete Gitterstruktur ein weiteres Ausreißen der Fenestrierungen bzw. Öffnungen verhindert.

Die Materialien, aus denen die Verstärkungselemente, insbesondere die Textilfäden und/oder die Klebstoffe bestehen, sind bspw. Polyester, Polymerschaum, UVhärtende Polymere.

Die erfindungsgemäße Gefäßprothese kann zwischen einem und acht Fenestrierungs-Bereiche, vorzugsweise, eins, zwei, drei, vier, fünf, sechs, sieben oder acht, Fenestrierung-Bereiche aufweisen, die sich jeweils zwischen zwei hintereinander angeordneten Ringen der Gefäßprothese befinden.

Ein Fenestrierungs-Bereich ist bei der erfindungsgemäßen Gefäßprothese dabei derart ausgestaltet und dimensioniert, dass im Fenestrierungs-Bereich zwischen einer und sechs, vorzugsweise eine, zwei, drei, vier oder fünf, Öffnungen/Fenestrierungen für Seitenäste geschaffen werden können.

Gemäß einer spezifischen Ausführungsform der erfindungsgemäßen intraluminalen Gefäßprothese ist deren Prothesenmaterial insgesamt aus Fenestrierungs-Bereichen besteht, welche eine weniger dichte Maschenstruktur aufweisen, sowie eine Gewebestruktur, die gitterartige Verstärkungselemente aufweist. Die Maschendichte dieser Ausführungsform ist dabei derart gewählt, dass die Fäden der Gewebestruktur durch entsprechende Dilatations-/Katheter-/Führgunsdraht-Elemente verdrängt werden können, und das Prothesenmaterial nicht verletzt wird.

Bei dieser Ausführungsform ist von Vorteil, dass es keine Rolle spielt, an welche Position des Gefäßes der Fenestrierungs-Bereich vorgeschoben wird, da die gesamte Gefäßprothese einen Fenestrierungs-Bereich besitzt, und daher über ihre gesamte Länge, bzw. zwischen sämtlichen vorliegenden Ringen, dazu geeignet ist, mit Öffnungen versehen zu werden.

Es versteht sich, dass dem Fachmann anhand der vorliegend bereitgestellten Lehre klar sein wird, dass die erfindungsgemäße Gefäßprothese an verschiedenen Stellen über ihre gesamte Länge und über den gesamten Umfang hinweg Fenestrierungs-Bereiche aufweisen kann, die in Abhängigkeit des zu behandelnden Gefäßes, bzw. in Abhängigkeit von vorliegenden abgehenden Gefäßen dimensioniert werden können.

Gemäß einer Ausführungsform der erfindungsgemäßen Gefäßprothese besitzt der zumindest eine Fenestrierungs-Bereich einen Durchmesser von ca. zwischen 2 bis 15 mm. Es versteht sich, dass auch die Durchmesser der Fenestrierungs-Bereiche mit Rücksicht auf den jeweils abgehenden Seitenast der Prothese bzw. auf das abgehende Blutgefäß dimensioniert sein werden.

Bei einem Vorliegen mehrerer Fenestrierungs-Bereiche können diese entsprechend in einer Ausführungsform der erfindungsgemäßen Gefäßprothese die gleichen oder unterschiedliche Durchmesser aufweisen.

Gemäß einer bevorzugten Ausführungsform weist die intraluminale Gefäßprothese einen Durchmesser von zwischen 5 mm und 70 mm auf, wobei bevorzugt ist, dass der Durchmesser zwischen 10 und 50 mm liegt.

Dem Fachmann wird allerdings klar sein, dass auch andere Bereiche möglich sind, und dass der letztendlich gewählte Durchmesser in Abhängigkeit des zu behandelnden Gefäßes gewählt werden wird.

Geeignete Materialien für die intraluminale Gefäßprothese sind für die hintereinander angeordneten Ringe ein selbstexpandierendes Material, beispielsweise Nitinol, sowie für das Prothesenmaterial insbesondere Polyester, Polyurethan, Polytetrafluoräthylen oder ultrahochmolekulargewichtiges Polyethylen (UHMPE), wobei Polyestergewebe bevorzugt ist. Dem Fachmann wird auch bezüglich der Materialien und unter Bezugsnahme auf die hierin offenbarte Lehre klar sein, dass auch andere, im Stand der Technik bekannte Materialien zum Einsatz kommen können, mithin also alle Materialien, die zur Ausbildung einer Gewebestruktur geeignet sind, und gleichzeitig dehnbare Maschen aufweisen.

Entsprechend betrifft die vorliegende Erfindung auch eine intraluminale Gefäßprothese, die neben dem hohlzylindrischen Grundkörper noch zumindest einen weiteren hohlzylindrischen Seitenkörper umfasst, welcher in die im Fenestrierungs-Bereich auszubildende zumindest eine Öffnung einbringbar ist.

Wie bereits einleitend erwähnt, können in die im Fenestrierungs-Bereich auszubildenden Öffnungen Seitenäste eingebracht werden, nachdem die intraluminale Gefäßprothese in das zu behandelnde Gefäß eingebracht und die Öffnungen ausgebildet wurden. Diese Seitenkörper werden durch die intraluminale Gefäßprothese und durch die Öffnung in das abgehende Seitengefäß eingeführt, um diesem den Blutfluss, der durch die intraluminale Gefäßprothese geleitet wird, aufrechtzuerhalten. Dabei ist bevorzugt, wenn diese zweiten Gefäßprothesen Metall-Stents ohne Prothesenmaterial oder aber ebenfalls Stentgrafts mit Prothesenmaterial darstellen.

Wie weiter oben erwähnt, kann die erfindungsgemäße Prothese auch verwendet werden in einem Verfahren zur Fenestrierung einer intraluminalen Gefäßprothese, das die folgenden Schritte aufweist:
- Bereitstellen einer erfindungsgemäßen intraluminalen Gefäßprothese,
- Durchdringen des Fenestrierungs-Bereiches der intraluminalen Gefäßprothese mit einem Erweiterungselement, insbesondere einem Katheter, Dilator, oder Führungsdraht, oder deren Spitzen, zur Ausbildung von zumindest einer Öffnung im Fenestrierungs-Bereich, wobei das Prothesenmaterial im Bereich der zu bildenden Öffnung durch das Erweiterungselement verdrängt wird.

Dieses Verfahren bietet den Vorteil, dass das Prothesenmaterial der Gefäßprothese nicht verletzt, durchstochen oder zerrissen werden muss, um Öffnungen in dem Fenestrierungs-Bereich auszubilden. Vielmehr können durch das Erweiterungselement bereits vorliegende Maschen der Gewebestruktur vergrößert werden, wodurch das Prothesenmaterial weder zerrissen noch durchstochen wird, und dadurch die Gefahr des weiteren Einreißens der Öffnung bzw. Fenestrierung vermieden wird.

Dabei wird vorliegend unter einem "Erweiterungselement" jedes Mittel verstanden, mit Hilfe dessen in dem Fenestrierungs-Bereich eine Öffnung/Fenestrierung geschaffen werden kann, ohne das Prothesenmaterial zu verletzen oder zu zerreißen. Geeignete Erweiterungselemente sind dabei bspw. ein Katheter, Dilator oder ein Führungsdraht, bzw. die Dilatoren-, Katheter- oder Führungsdrahtspitze.

Dilatoren und Führungsdrähte sind im Stand der Technik hinreichend bekannt und werden bereits seit längerem im Zusammenhang mit Gefäßprothesen eingesetzt, so dass dem Fachmann klar sein wird, welche Mittel er erfindungsgemäß einsetzen kann.

Die erfindungsgemäße Prothese kann ferner verwendet werden in einem Verfahren zur Einbringung einer intraluminalen Gefäßprothese, wobei das Verfahren die folgenden Schritte aufweist:
- Einbringen und Freisetzen einer ersten erfindungsgemäßen intraluminalen Gefäßprothese in ein Blutgefäß eines Patienten,
- Ausbilden von zumindest einer Öffnung im Fenestrierungs-Bereich des hohlzylindrischen Grundkörpers durch Verdrängen des Prothesenmaterials im Fenestrierungs-Bereich des Prothesenmaterials, bzw. durch Aufweiten bereits vorliegender Maschen im Fenestrierungs-Bereich des Prothesenmaterials,
- ggf. Einbringen einer zweiten Gefäßprothese durch die Öffnung im Fenestrierungs-Bereich des hohlzylindrischen Grundkörpers zur Ausbildung von zumindest einem Seitenast der intraluminalen Gefäßprothese.

Mit diesem Verfahren kann erstmals die Möglichkeit geschaffen werden, eine Gefäßprothese in ein Gefäß zum Zwecke der Ausbildung von zumindest einer Fenestrierung/Öffnung einzuführen, ohne dass die genaue Rotations-Position der Gefäßprothese in dem Gefäß berücksichtigt werden müsste. Der chirurgische Eingriff wird dadurch wesentlich erleichtert und beschleunigt.

Um die erfindungsgemäße Gefäßprothese in das zu behandelnde Gefäß einzubringen, können im Stand der Technik bekannte oder übliche Einführsysteme mit Katheter, Rückzugshülle und Pusher eingesetzt werden. Auch diese sind dem Fachmann bekannt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung eines Ausschnitts einer erfindungsgemäßen intraluminalen Gefäßprothese;
- Fig. 2: die in Fig. 1 dargestellte Ausführungsform der intraluminalen Gefäßprothese, mit eingeführtem Seitenast;
- Fig. 3a: zwei in die Gefäße eingebrachte Ausführungsbeispiele der intraluminalen Gefäßprothese, wie in Fig. 1 und 2 gezeigt, wobei diese Ausführungsbeispiele im Bereich der Aorten-Bifurkation eingeführt sind;
- Fig. 3b: eine schematische Darstellung einer erfindungsgemäßen intraluminalen Gefäßprothese, die mit Seitenästen in den Aortenbogen eingebracht ist; und
- Fig. 3c: eine schematische Darstellung einer noch weiteren Ausführungsform der intraluminalen Gefäßprothese, eingebracht in ein weiteres Gefäß eines Patienten, mit vier abgehenden Seitenästen der Gefäßprothese.

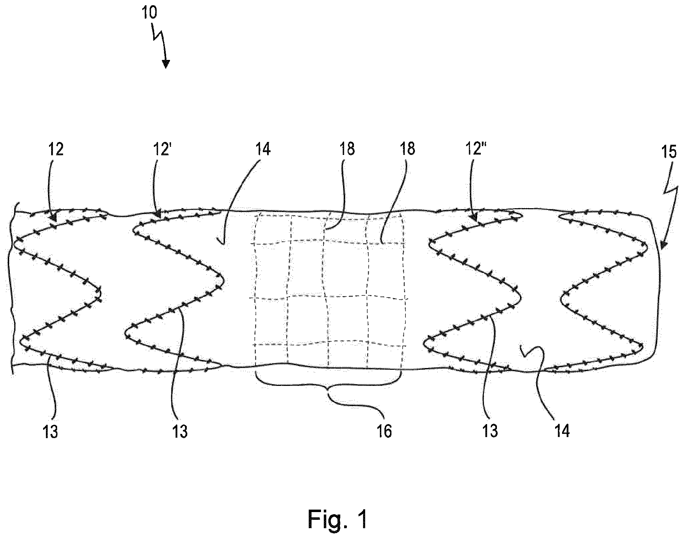

In Fig. 1 ist mit 10 insgesamt eine intraluminale Gefäßprothese bezeichnet, mit in ihrer Längsrichtung hintereinander angeordneten Ringen 12 aus mäanderförmig umlaufenden Stützen 13 und einem an den Ringen 12 befestigten und diese verbindenden Prothesenmaterial 14. Zwischen dem Ring 12' und 12" ist ein Fenestrierungs-Bereich 16 vorgesehen, der in dem in Fig. 1 gezeigten Ausführungsbeispiel durch eine umgreifende Klammer angezeigt ist. In diesem Fenestrierungs-Bereich weist das Prothesenmaterial eine Gewebestruktur auf, die unterschiedlich ist zu den Bereichen des Prothesenmaterials, welche bei dem in Fig. 1 gezeigten Ausführungsbeispiel rechts und links von dem Fenestrierungs-Bereich 16 liegen. Der Fenestrierungs-Bereich 16 weist eine geringere Maschendichte auf sowie gitterartige Verstärkungselemente 18, die im Fenestrierungs-Bereich 16 in das Prothesenmaterial 14 eingewebt, oder aufgeklebt sind.

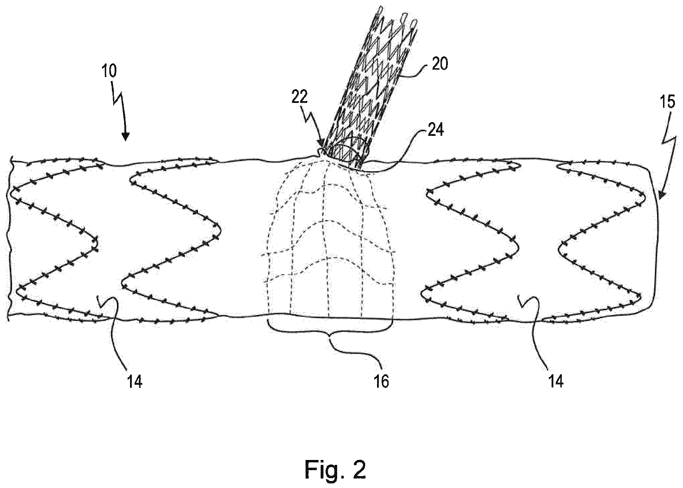

Fig. 2 zeigt die in Fig. 1 gezeigte Ausführungsform der erfindungsgemäßen intraluminalen Gefäßprothese 10, wobei hierdurch den hohlzylindrischen Grundkörper 15 ein Seitenast oder eine Seitenprothese 20 vorgesehen ist, der durch eine im Fenestrierungs-Bereich 16 vorgesehene Öffnung 22 durchgeschoben wurde. Bei der in Fig. 2 gezeigten Ausführungsform der Seitenprothese handelt es sich um einen Stent, der bei dieser Ausführungsform kein Prothesenmaterial aufweist. Es versteht sich, dass auch andere Ausführungsformen für Seitenäste/-prothesen durch die im Fenestrierungs-Bereich 16 gebildeten Öffnungen 22 hindurchgeschoben werden können, deren Ausgestaltungen und Dimensionen dem Fachmann ausgehend von der hierin offenbarten Lehre klar sein werden.

Fig. 2 ist zu entnehmen, dass die Öffnung 22, die im Fenestrierungs-Bereich 16 gebildet ist, durch eine Gitterstruktur bzw. ein Gitterloch 24 begrenzt wird, wodurch eine Aufweitung und ein Ausreißen der Öffnung 22 nach Einführen der weiteren Gefäßprothese 20 verhindert.

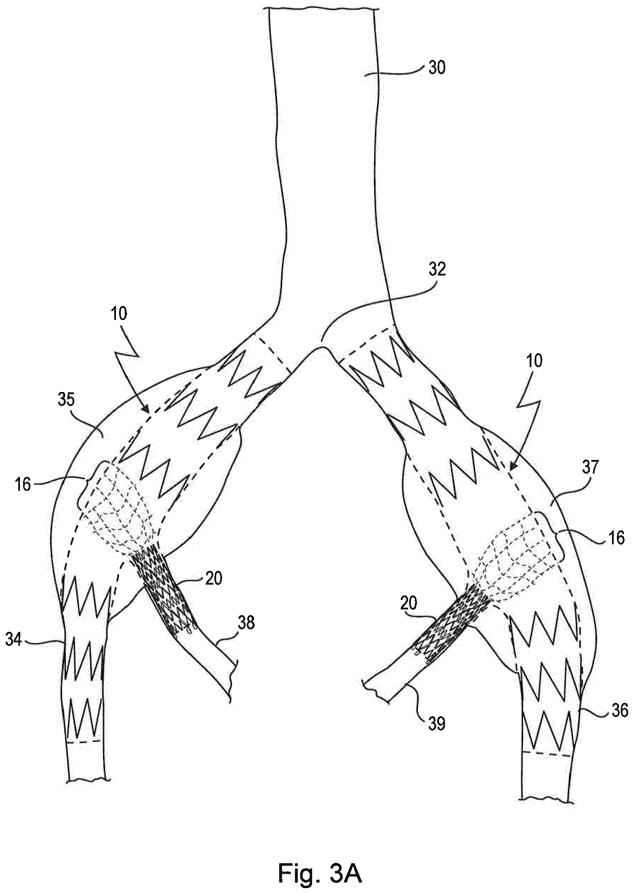

In den Fig. 3a bis 3c werden nun Ausführungsbeispiele für in Gefäße eingeführte erfindungsgemäße intraluminale Gefäßprothesen beschrieben.

In Fig. 3a ist gezeigt, wie die in Fig. 1 dargestellte Ausführungsform der erfindungsgemäßen intraluminalen Gefäßprothese 10 in der Aorten-Bifurkation positioniert ist. Dabei ist mit 30 insgesamt die Aorta bezeichnet, wobei das Bezugszeichen 32 die Aortengabel (Bifurcatio aortae) angibt. An der Aortengabel 32 teilt sich die Aorta 30 in die beiden Arteriae iliacae 34 und 36, die jeweils in das linke und rechte Bein hinabführen. Im jeweils oberen Bereich der Arteriae iliacae 34, 36 befindet sich ein Aneurysma 35 bzw. 37, das jeweils durch Einbringung einer erfindungsgemäßen intraluminalen Gefäßprothese 10 überbrückt worden ist.

Wie Fig. 3a zu entnehmen ist, wurde die intraluminale Gefäßprothese 10 jeweils derart in die Arteria iliaca 34, 36 eingeführt bzw. positioniert und freigesetzt, dass der Fenestrierungs-Bereich 16 im Bereich eines abgehenden Gefäßes 38 bzw. 39 zu liegen kommt. Ferner weist die erfindungsgemäße intraluminale Gefäßprothese 10 jeweils eine durch den Fenestrierungs-Bereich 16 geführte Seitenprothese 20 auf, die in das Seitengefäß 38 bzw. 39 gelegt ist.

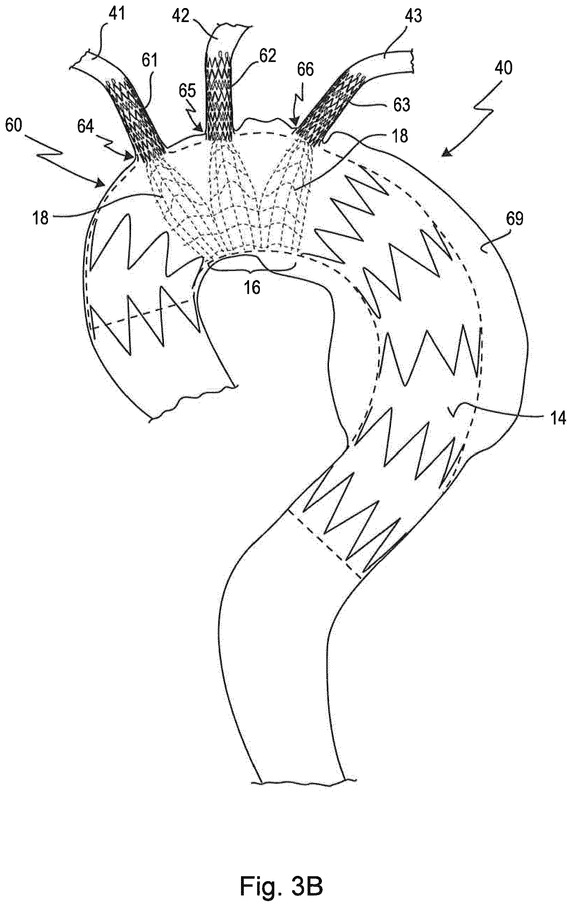

Fig. 3b zeigt eine weitere Ausführungsform 60 der erfindungsgemäßen intraluminalen Gefäßprothese, wobei bei der in Fig. 3b gezeigten Ausführungsform gleiche Merkmale wie bei der in Fig. 1 gezeigten Ausführungsform mit gleichen Bezugszeichen versehen sind. Auch die Gefäßprothese 60 weist hintereinander angeordente mäanderförmig umlaufende Ringe 12 auf, mit einem daran angebrachten Prothesenmaterial 14.

Ferner weist die in Fig. 3b gezeigte intraluminale Gefäßprothese 60 einen Fenestrierungs-Bereich 16 auf, in welchem Öffnungen 64, 65, 66 vorgesehen sind, durch welche Seitenäste/-prothesen 61, 62, 63 angebracht sind. Das in Fig. 3b gezeigte Ausführungsbeispiel ist dabei im Aortenbogen 40 eingebracht, wobei mit 41, 42 und 43 die abgehenden Gefäße des Truncus brachiocephalicus, der Arteria carotis communis und der Arteria subclavia bezeichnet sind. Die im Fenestreirungsbereich 16 ebenfalls vorgesehenen gitterartigen Verstärkungselemente 18 sind in Fig. 3b gestrichelt dargestellt.

Auch bei der in Fig. 3b gezeigten Darstellung des Aortenbogens 40 ist mit 64 ein Aneurysma 64 bezeichnet, das durch die intraluminale Gefäßprothese 60 überbrückt wird. Es versteht sich, dass die intraluminale Gefäßprothese 60 insbesondere in diesem Bereich des Aneurysmas 64 blutdicht ist.

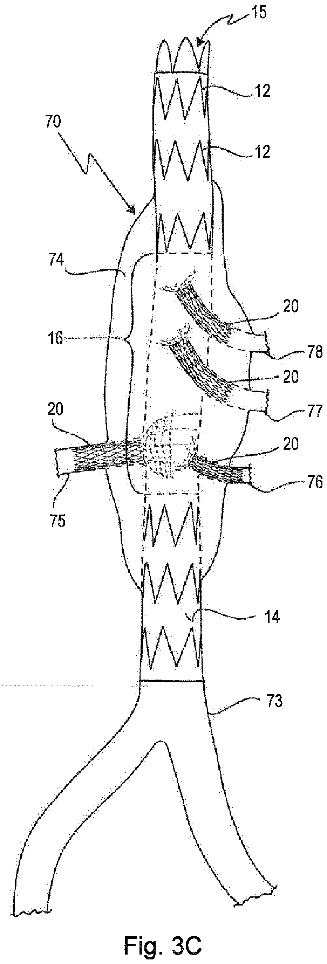

Fig. 3c zeigt schließlich eine weitere Ausführungsform 70 der erfindungsgemäßen Gefäßprothese70, mit hintereinander angeordneten mäanderförmig umlaufenden Ringen 12, und mit einem Fenestrierungs-Bereich 16 zwischen den Ringen 12'" und 12"", wobei hier die gitterförmigen Verstärkungselemente 18 aus Gründen der Übersichtlichkeit nur schematisch und nur teilweise eingezeichnet bzw. gestrichelt sind.

Bei der in Fig. 3c gezeigten Ausführungsform 70 sind vier Seitenäste/- prothesen 20 vorgesehen, die im Bereich eines Aneurysmas 74 in abzweigenden Seitengefäße 75, 76, 77 und 78 hineinreichen.

In Fig. 3c ist gut zu erkennen, dass die drei Seitenäste 20 der Gefäßprothese 70 auf dem Umfang und der Länge der Gefäßprothese 70 verteilt bzw. versetzt vorgesehen sein können, und zwar je nach Lage der abgehenden und zur versorgenden Seitengefäße. Die in Fig. 3c gezeigte Ausführungsform wurde - wie auch die in den Flg. 3a und 3b gezeigten Ausführungsformen - wie folgt in das Gefäß 73 eingebracht: Zunächst wird der hohzylindrische Grundkörper 15 im komprimierten Zustand in das Gefäß eingeführt und an der erwünschten Position zur Überbrückung des Aneurysmas 74 positioniert, derart, dass der Fenestrierungs-Bereich im Bereich der vom Gefäß abgehenden Seitengefäße zu liegen kommt. Nach der Freisetzung des hohlzylindrischen Körper 15 wird mittels geeigneter Erweiterungselemente im Fenestrierungs-Bereich zumindest eine Öffnung/Fenestrierung geschaffen, deren Anzahl von den zu versorgenden, abgehenden Seitengefäßen abhängt, und anschließend Seitenäste/-prothesen 20 durch diese Öffnungen vorgeschoben - je nach Möglichkeit/Notwendigkeit von innen oder von außen - zur Schaffung von Seitenästen der Gefäßprothese 10, 60, 70.

Die Gefäßprothese 10, 60, 70 kann hierfür, entweder am hohlzylindrischen Grundkörper 15 und/oder an dem Seitenast/den Seitenästen 20 bspw. röntgendichte Marker (nicht dargestellt) aufweisen, die das Einführen und Positionieren der Gefäßprothese 10, 60, 70 erleichtern.

Die in den Figuren 1 bis 3 gezeigte erfindungsgemäße Gefäßprothese kann ferner auch direkt im Bereich der Aorten-Bifurkation (siehe Fig. 3c) eingesetzt werden. Der Fenestrierungs-Bereich wird dabei derart in der Bifurkation der Aorta platziert, dass durch die Öffnung, die im Fenestrierungs-Bereich gebildet wird, ein Seitenast die Arteria iliaca gelegt werden kann. Der hohlzylindrische Grundkörper der Gefäßprothese erstreckt sich dabei von der Arteria iliaca über die Bifurcatio Aortae in die Aorta.

Die in den Figuren 3a bis 4c gezeigten Ausführungsformen wurden wie folgt in die betreffenden Gefäße eingebracht: Zunächst wird der hohlzylindrische Grundkörper 15 im komprimierten Zustand in das zu behandelnde Gefäß eingeführt und an der erwünschten Position zur Überbrückung des Aneurysmas positioniert, und zwar derart, dass der/die Fenestrierungs-Bereich(e) (16) im Bereich der/des vom Gefäß abgehenden Seitengefäße(s) zu liegen kommt. Nach der Freisetzung des hohlzylindrischen Körpers 15 wird mittels geeigneter Erweiterungselemente in den/dem Fenestrierungs-Bereich(en) 16 jeweils eine Öffnung/Fenestrierung 22 geschaffen, und anschließend Seitenäste/- prothesen durch diese Öffnungen vorgeschoben - je nach Möglichkeit/Notwendigkeit von innen oder von außen - zur Schaffung von Seitenästen der Gefäßprothese.

Die Gefäßprothese 10, 60, 70 kann hierfür, entweder am hohlzylindrischen Grundkörper 15, an den jeweiligen Fenestrierungs-Bereichen 16, und/oder an dem Seitenast/den Seitenästen bspw. röntgendichte Marker (nicht dargestellt) aufweisen, die das Einführen und Positionieren der Gefäßprothese 10, 60, 70 erleichtern. Wenn bei der erfindungsgemäßen Prothese mehrere Fenestrierungs-Bereiche vorgesehen sind, so können diese bspw. unterschiedliche Marker aufweisen.

## Patentansprüche

1. Intraluminale Gefäßprothese (10; 60; 70) zur Implantation in ein Blutgefäß, mit in ihrer Längsrichtung hintereinander angeordneten Ringen (12) aus mäanderförmig umlaufenden Stützen (13) und einem an den Ringen (12) befestigten und diese verbindenden Prothesenmaterial (14) mit einer Gewebestruktur, welches einen hohlzylindrischen Grundkörper (15) mit umfänglich geschlossenem Mantel bildet, wobei die Gewebestruktur des Prothesenmaterials (14) aus miteinander verwobenen Fäden gebildet ist und eine Maschendichte aufweist, wobei
zwischen zwei hintereinander angeordneten Ringen (12) der Gefäßprothese (10; 60; 70) im Prothesenmaterial (14) zumindest ein Fenestrierungs-Bereich (16) für die Ausbildung von zumindest einer Öffnung (22) für von dem hohlzylindrischen Grundkörper (15) abgehende Seitenäste (20; 61, 62, 63) vorgesehen ist, welcher Fenestrierungs-Bereich (16) eine zu dem übrigen Prothesenmaterial (14) unterschiedliche Gewebestruktur aufweist, wobei die Gewebestruktur in dem Fenestrierungs-Bereich (16) eine geringere Maschendichte aufweist als in dem übrigen Prothesenmaterial (14), sowie gitterartige Verstärkungselemente (18), **dadurch gekennzeichnet, dass** die gitterartigen Verstärkungselemente durch in dem Fenestrierungs-Bereich (16) eingewebte Textilfäden (18) oder aufgebrachte Klebstoffe bewirkt und nur im Fenestrierungs-Bereich (16) vorgesehen sind.

2. Intraluminale Gefäßprothese (10; 60; 70) nach Anspruch 1, **dadurch gekennzeichnet, dass** die Gewebestruktur des Prothesenmaterials (14) im Fenestrierungs-Bereich (16) durch eine integrierte Webung hergestellt ist und das Prothesenmaterial (14) einstückig gewebt und ausgebildet ist.

3. Intraluminale Gefäßprothese (10; 60; 70) nach Anspruch 1 oder 2, **dadurch gekennzeichnet, dass** der Fenestrierungs-Bereich (16) sich über den vollen Umfang des hohlzylindrischen Grundkörpers (15) erstreckt.

4. Intraluminale Gefäßprothese (10; 60; 70) nach einem der Ansprüche 1 bis 3, **dadurch gekennzeichnet, dass** im Fenestrierungs-Bereich (16) in die Gewebestruktur gitterartig angebrachte Textilfäden (18) zur Ausbildung der gitterartigen Verstärkungselemente (18) vorgesehen sind.

5. Intraluminale Gefäßprothese (10; 60; 70) nach einem der Ansprüche 1 bis 4, **dadurch gekennzeichnet, dass** der Fenestrierungs-Bereich (16) über die Länge des hohlzylindrischen Grundkörpers (15) derart dimensioniert ist, dass er für die Ausbildung von Öffnungen für zwischen einem bis sechs Seitenästen (20; 61, 62, 63) des hohlzylindrischen Grundkörpers geeignet ist.

6. Intraluminale Gefäßprothese (10; 60; 70) nach einem der Ansprüche 1 bis 5, **dadurch gekennzeichnet, dass** sie einen Durchmesser von zwischen 5 mm und 70 mm aufweist.

7. Intraluminale Gefäßprothese (10; 60; 70) nach einem der Ansprüche 1 bis 6, **dadurch gekennzeichnet, dass** sie neben dem hohlzylindrischen Grundköper (15) ferner zumindest einen hohlzylindrischen Seitenkörper (20; 61, 62, 63) umfasst, welcher in die im Fenestrierungs-Bereich (16) auszubildende zumindest eine Öffnung (22) einbringbar ist.

## Claims

1. Intraluminal vascular prosthesis (10; 60; 70) for implantation in a blood vessel, having rings (12), which are successively arranged in its longitudinal direction, and which have circumferential meandering struts (13) and a prosthesis material (14), which is fixed to the rings (12) and connects them, the prosthesis material (14) having a fabric structure, which forms a hollow cylindrical body (15) with a circumferentially closed mantle, wherein the fabric structure of the prosthesis material (14) is formed of threads interwoven with one another and has a mesh density, wherein, in the prosthesis material (14), between two successively arranged rings (12) of the vascular prosthesis (10; 60; 70) at least one fenestration area (16) is provided for forming of at least one opening (22) for side branches (20; 61, 62, 63) branching off from the hollow cylindrical body (15), which fenestration area (16) has a fabric structure being different from the fabric structure of the remaining prosthesis material (14), wherein in the fenestration area, the fabric structure has a lesser mesh density than in the remaining prosthesis material (14), as well as lattice-like reinforcement elements (18), **characterized in that** the lattice-like reinforcement elements (18) are effected by textile threads woven into the fenestration area (16) or by applied adhesives, and are provided in the fenestration area (16) only.

2. The intraluminal vascular prosthesis (10; 60; 70) of claim 1, **characterized in that** the fabric structure of the prosthesis material (14) in the fenestration area (16) is made by integrated weaving and that the prosthesis material (14) is woven and formed integrally.

3. The intraluminal vascular prosthesis (10; 60; 70) of claim 1 or 2, **characterized in that** the fenestration area (16) extends over the complete circumference of the hollow cylindrical body (15).

4. The intraluminal vascular prosthesis (10; 60; 70) of any of claims 1 to 3, **characterized in that**, in the fenestration area (16), lattice-like attached textile threads (18) are provided in the fabric structure for forming the lattice-like reinforcement elements (18).

5. The intraluminal vascular prosthesis (10; 60; 70) of any of claims 1 to 4, **characterized in that** the fenestration area (16), over the length of the hollow cylindrical body (15), is dimensioned such, that it is suitable for forming of openings for between one to six side branches (20; 61, 62, 63) of the hollow cylindrical body.

6. The intraluminal vascular prosthesis (10; 60; 70) of any of claims 1 to 5, **characterized in that** it has a diameter of between 5 mm and 70 mm.

7. The intraluminal vascular prosthesis (10; 60; 70) of any of claims 1 to 6, **characterized in that** it comprises, besides the hollow cylindrical body (15), at least one hollow cylindrical side branch (20; 61, 62, 63), which is introduceable into the at least one opening (22) to be formed in the fenestration area (16).

## Revendications

1. Prothèse vasculaire intraluminale (10; 60; 70) destinée à être implantée dans un vaisseau sanguin, avec des anneaux (12) composés de segments périphériques sinueux (13) disposés l'un derrière l'autre dans sa direction longitudinale et une matière de prothèse (14) attachée aux anneaux (12) et reliant ceux-ci avec une structure tissulaire, qui forme un corps de base cylindrique creux (15) avec une enveloppe fermée en périphérie, dans laquelle la structure tissulaire de la matière de prothèse (14) est formée de fils entrelacés les uns avec les autres et présente une densité de mailles, dans laquelle il est prévu entre deux anneaux (12) de la prothèse vasculaire (10; 60; 70) disposés l'un derrière l'autre dans la matière de prothèse (14) au moins une région de fenestration (16) pour la formation d'au moins une ouverture (22) pour des branches latérales (20; 61, 62, 63) partant du corps de base cylindrique creux (15), ladite région de fenestration (16) présentant une structure tissulaire différente du reste de la matière de prothèse (14), dans laquelle la structure tissulaire dans la région de fenestration (16) présente une densité de mailles plus faible que dans le reste de la matière de prothèse (14), ainsi que des éléments de renforcement en forme de grille (18), **caractérisée en ce que** les éléments de renforcement en forme de grille sont formés par des fils textiles (18) tissés ou par des matières adhésives appliquées dans la région de fenestration (16) et ne sont prévus que dans la région de fenestration (16).

2. Prothèse vasculaire intraluminale (10; 60; 70) selon la revendication 1, **caractérisée en ce que** la structure tissulaire de la matière de prothèse (14) dans la région de fenestration (16) est produite par un tissage intégré et la matière de prothèse (14) est tissée et formée en une seule pièce.

3. Prothèse vasculaire intraluminale (10; 60; 70) selon la revendication 1 ou 2, **caractérisée en ce que** la région de fenestration (16) s'étend sur toute la périphérie du corps de base cylindrique creux (15).

4. Prothèse vasculaire intraluminale (10; 60; 70) selon l'une quelconque des revendications 1 à 3, **caractérisée en ce qu'**il est prévu dans la région de fenestration (16) des fils textiles (18) installés en forme de grille dans la structure tissulaire pour la réalisation des éléments de renforcement en forme de grille (18).

5. Prothèse vasculaire intraluminale (10; 60; 70) selon l'une quelconque des revendications 1 à 4, **caractérisée en ce que** la région de fenestration (16) est dimensionnée sur la longueur du corps de base cylindrique creux (15) de telle manière qu'elle convienne pour la formation d'ouvertures pour entre une et six branches latérales (20; 61, 62, 63) du corps de base cylindrique creux.

6. Prothèse vasculaire intraluminale (10; 60; 70) selon l'une quelconque des revendications 1 à 5, **caractérisée en ce qu'**elle présente un diamètre compris entre 5 mm et 70 mm.

7. Prothèse vasculaire intraluminale (10; 60; 70) selon l'une quelconque des revendications 1 à 6, **caractérisée en ce qu'**elle comprend en outre, en plus du corps de base cylindrique creux (15), au moins un corps latéral cylindrique creux (20; 61, 62, 63), qui peut être introduit dans au moins une ouverture (22) à pratiquer dans la région de fenestration (16).
